# EUROPEAN PATENT SPECIFICATION

(11) **EP 4 135 813 B1**
(45) Date of publication and mention of the grant of the patent: **06.08.2025**
(21) Application number: 20720018.9
(22) Date of filing: 16.04.2020
(51) Int. Cl.: A61M 16/08, A61M 16/12, A61M 16/00

(54) **DEVICE AND SYSTEM FOR RESPIRATORY SUPPORT**
VORRICHTUNG UND SYSTEM ZUR ATEMUNTERSTÜTZUNG
DISPOSITIF ET SYSTÈME D'ASSISTANCE RESPIRATOIRE

(43) Date of publication of application: 22.02.2023
(73) Proprietor: Neores AB, 831 45 Östersund (SE)
(72) Inventor: DREVHAMMAR, Thomas, 831 43 Östersund (SE); NILSSON, Kjell, 831 45 Östersund (SE)
(74) Representative: AWA Sweden AB
(86) International application number: PCT/EP2020/060773
(87) International publication number: WO 2021/209141

(56) References cited:
- EP-A1- 0 512 285
- EP-A1- 2 231 244
- WO-A1-2012/108826
- WO-A1-2013/067164
- WO-A1-2018/236228
- US-A1- 2009 250 059
- US-A1- 2009 253 995
- US-B1- 6 915 705

## Description

### FIELD OF THE INVENTION

The present invention relates to a device and a system for positive pressure ventilation (PPV) and continuous positive airway pressure (CPAP) treatment for e.g. neonatal resuscitation and initial respiratory support.

### BACKGROUND OF THE INVENTION

Positive pressure ventilation (PPV) and continuous positive airway pressure (CPAP) are two types of mechanical respiratory support commonly used for resuscitation and stabilisation of newborn infants. Which of the two respiratory support systems to use depends on whether the infant is breathing or not. While PPV is used for the non-breathing infant, CPAP is used for the breathing infant. An infant who is not breathing after birth should be ventilated. This can be accomplished with PPV using e.g. a face mask, or an endotracheal tube. In the majority of cases a mask is used. If the infant starts to breathe or was breathing after birth, support of ventilation using CPAP is the recommended treatment for several conditions. After return of spontaneous breathing some infants will still need PPV intermittently if they do not breathe adequately or stop breathing. This is particularly common when treating premature neonates. Thus, both types of support are common and often the need changes during the resuscitation period. During both PPV and CPAP the distending pressure helps the lung to maintain aeration. This is referred to as positive end expiratory pressure (PEEP) when providing PPV and is, for this type of device, functionally equivalent to CPAP.

A device and system which can provide both positive pressure ventilation and continuous positive airway pressure is disclosed in WO2012/108826. The device comprises a first fresh gas flow tube arranged to provide a first fresh gas flow, a second fresh gas flow tube, a variable flow CPAP generator, a connector connectable to any infant interface, and a pressure release valve. The variable flow CPAP generator comprises a first connection portion to which the first fresh gas flow tube is connected, a second connection portion to which the infant interface connector is connected, and a third connection portion which is an outlet of the variable flow CPAP generator having an open end. The second fresh gas flow tube bypasses the variable CPAP generator and is arranged to provide a second fresh gas flow which is added to the first fresh gas flow in the positive pressure ventilation mode when the open end of the outlet is occluded. Thereby, in the PPV mode, the pressure is increased at a higher rate than if only the first fresh gas flow had been used which allows for a shortened inspiratory rise time. The system can be used for non-invasive ventilation and provides a low imposed work of breathing for the breathing child treated with CPAP and an easy switch between PPV and CPAP respiratory support without change of equipment.

For an optimal functioning of the system it is necessary to adjust the fresh gas flow supplied during resuscitation through the use of the second fresh gas flow. Adding the second fresh gas flow in the PPV mode provides an adjustable inspiratory rise time, which e.g. can be shortened by increasing the second fresh gas flow to the second fresh gas flow tube. This is desirable to provide a stable and efficient ventilation. However, applying the second fresh gas flow is not possible in all medical installations, due to e.g. lack of outputs at the gas source for connecting the second fresh gas flow tube. Further, the second fresh gas flow increases the complexity of the device and may increase the risk of user errors with potentially serious consequences. Consequently, making use of a second fresh gas flow through the second fresh gas flow tube is not always possible, resulting in a less efficient resuscitation treatment.

Therefore, there is a need within the technical field of devices providing both PPV and CPAP to overcome the problems that exist today.

The following documents are also known in the art, namely EP 0 512 285 A1, EP 2 231 244 A1, WO 2013/067164 A1, WO 2018/236228 A1, US 2009/253995 A1, US 6 915 705 B1 and US 2009/250059 A1.

### SUMMARY OF THE INVENTION

The invention is defined in the appended claims. It is an object of the present disclosure to provide a device for PPV and CPAP treatment that alleviates the above-mentioned problems. The object is achieved by a device for PPV and CPAP treatment according to the present disclosure as disclosed herein.

Thus, in accordance with an aspect of the present disclosure, there is provided a device for positive pressure ventilation and continuous positive airway pressure treatment comprising:
- a fresh gas flow inlet, arranged to receive a fresh gas flow through a fresh gas flow tube connectable thereto;
- a patient interface end, arranged to be connected with a patient interface;
- an outlet having an open end;
- a variable flow CPAP generator comprising first, second and third connection portions, wherein the first connection portion is connected with the fresh gas flow inlet, the second connection portion is connected with the patient interface end; and the third connection portion is connected with the outlet, wherein the generated CPAP level is adjusted by varying the fresh gas flow to the variable flow CPAP generator; and
- an internal leakage channel arranged to extend between the fresh gas flow inlet and at least one of the patient interface end and the outlet, such to create an internal fresh gas leakage flow there between, bypassing the first connection portion of the variable flow CPAP generator, which internal fresh gas leakage flow is added to the fresh gas flow provided by the variable flow CPAP generator in the PPV mode.

This provides a simplified device for CPAP and PPV treatment, particularly intended for resuscitation and stabilisation of infants. The device is easy to use and allows rapid switch between PPV and CPAP without change of equipment. A safe and quick switch between these types of respiratory support will improve care for an unstable child and lead to higher quality in resuscitation with reduced mortality and morbidity. Due to the internal leakage channel, the inspiratory flow in the PPV mode is increased, such that when the outlet is occluded the internal fresh gas leakage flow adds to the fresh gas flow provided by the variable flow CPAP generator, which rapidly increases the pressure and generates an inspiration of the patient.

The provision of an internal leakage channel simplifies gas supply to the device, which is only needed for the fresh gas flow inlet. Thus, one sole driver flow of fresh gas is needed for the device, i.e. for the one sole fresh gas flow inlet. This further allows for a reduction in size of the device with respect to when two driver flows are required, thereby also facilitating the handling and use of the same. A further advantage with providing only one driver flow for the device as herein disclosed is that it allows integration of the device in both low tech and high-tech ventilator equipment, requiring only one gas output channel for fresh gas supply from the ventilator equipment. That is, the device can be installed in a system comprising just one gas output channel for fresh gas supply. This allows integration of the device with existing CPAP drivers and ventilators without any modification of the hardware. Additionally, another advantage is that providing humidification of the flow to the fresh gas flow inlet is simple, as opposed to for a device comprising two driver flows.

The driver flow, i.e. the fresh gas flow supplied to the fresh gas flow inlet, can be set to an amount that allows for a desired CPAP to be generated by the variable flow CPAP generator. Due to the internal fresh gas leakage, when the open end of the outlet of the device is occluded, the internal fresh gas leakage flow is added to the fresh gas flow provided by the CPAP generator resulting in an increased flow for the inflation breath. The increased flow resulting from the occlusion of the outlet, corresponding to the PPV mode, provides for a decreased rise time of the pressure which is advantageous.

A series of devices can be provided, each of which is arranged to give a certain level of CPAP for a given range of supplied fresh gas flow. In order to achieve different levels of CPAP for a certain range of supplied fresh gas flows, the diameter of the internal leakage channel can be modified between devices, thus adjusting the amount of leakage generated by a certain device for a given range of supplied fresh gas flow. This allows providing devices for CPAP and PPV treatment suitable for a range of patients, from neonates to children of e.g. up to 10 kg of weight. Generally, the diameter of the internal leakage channel may be between 0,5 and 2 mm. In an embodiment of the device, the diameter of the internal leakage channel is 0,75 mm.

For purposes of this invention, the words "infant" and "child" are intended to encompass a patient such as a newborn, and a neonatal child which is in need of neonatal resuscitation and initial respiratory support. The words are also intended to encompass children and toddlers of up to around 10 kg of weight. The fresh gas flow is adapted to the weight and size of the child such that a desirable rise time is attained. For purposes of this disclosure, the wording "fresh gas flow" is air, oxygen or a mixture of these that flows through the system and its parts, and the wording "fresh gas flow tube" is wherein the fresh gas flows.

For purposes of this disclosure, the internal fresh gas leakage flow could be regarded as a bypass fresh gas flow, since the fresh gas leakage flow does not drive the variable flow CPAP generator as does the fresh gas flow. That is, in a sense, the fresh gas leakage flow bypasses the variable flow CPAP generator, at least the first connection portion thereof. Thereby, the internal fresh gas leakage flow does not contribute to the generation of the CPAP level of the device when used in the CPAP mode, which is solely generated by the variable flow CPAP generator and the fresh gas flow there through.

For purposes of this disclosure, the wording "variable flow CPAP generator" is a device intended to encompass any continuous positive airway pressure device where the CPAP level is adjusted by varying the fresh gas flow.

For the purposes of this disclosure, the wording "driver flow" will be used as a synonym to the fresh gas flow provided to the fresh gas flow inlet of the device.

For purposes of this disclosure, the wording "imposed work of breathing" is the additional work required to breathe through a device and is an established way of measuring the pressure stability of a system. Imposed work of breathing is obtained by calculating the area within a pressure-volume loop for one breath.

For purposes of this disclosure, the word "patient interface" is intended to encompass any interface that is suitable for connecting to a patient, e.g. an infant or child, such as a pair of nasal prongs, a mask, an endotracheal tube or any other suitable device.

There are different ways of arranging the internal fresh gas leakage channel in order to achieve the advantages set forth above.

In accordance with an embodiment of the device, the internal leakage channel comprises a hole through an internal wall portion of the device. The inventors have surprisingly found that providing a hole through an internal wall portion such that an internal leakage channel extends between the fresh gas flow inlet and one of the patient interface end and the outlet of the device for CPAP and PPV treatment, bypassing the first connection portion of the variable flow CPAP generator, generates an internal leakage fresh gas flow which does not alter the efficiency of the variable flow CPAP generator but allows for the advantages described herein.

In accordance with an embodiment of the device, the internal leakage channel partially extends through a portion of the second connection portion of the variable flow CPAP generator to the patient interface end of the device. In this embodiment, a hole is provided through an internal wall portion which extends between the fresh gas flow inlet and the second connection portion of the variable flow CPAP generator. In other words, the internal wall portion separates the fresh gas flow inlet from the second connection portion. Thereby, the internal leakage channel extends between the fresh gas flow inlet and the patient interface end through at least a portion of the second connection portion.

In accordance with an embodiment of the device, the internal leakage channel partially extends through a portion of the third connection portion of the variable flow CPAP generator to the outlet of the device. In this embodiment, a hole is provided through an internal wall portion which extends between the fresh gas flow inlet and the third connection portion of the variable flow CPAP generator, such that the internal leakage channel, made up of the hole and at least a portion of the third connection portion of the variable flow CPAP generator, extends between the fresh gas flow inlet and the outlet of the device.

In accordance with an embodiment of the device, the device further comprises a pressure release connection portion, arranged partially in parallel with the second connection portion of the variable flow CPAP generator, and having a first end connected with the patient interface end of the device and a second end connectable with a pressure release tube, wherein the internal leakage channel partially extends through the pressure release connection portion to the patient interface end of the device. The pressure release connection portion is generally tubular and extends through a portion of the device between the patient interface end and a side wall of the device, forming an internal passage there through. The pressure release tube is connectable with the hole through the side wall provided by the pressure release connection portion. In this embodiment, a hole is further provided through an internal wall portion which extends between the fresh gas flow inlet and the pressure release connection portion, separating the two, such that the internal leakage channel extends between the fresh gas flow inlet and the patient interface end of the device, bypassing the first connection portion of the variable flow CPAP generator.

In accordance with an embodiment of the device, the fresh gas flow in the device supplied to the fresh gas flow inlet is in the range of 5-15 liters per minute. Typically, the fresh gas flow provided is between 10 and 12 liters per minute. However, a fresh gas flow above 15 liters per minute is also possible to provide for treatment of an older child. The device is further configured such that this supplied fresh gas flow generates a level of CPAP of 3-10 cm H₂O. In a preferred embodiment, the generated level of CPAP is 5-6 cm H₂O.

The different embodiments of the device can be summarized as a device comprising a fresh gas flow inlet connected to a variable flow CPAP generator in turn connected to a patient interface end and to an outlet of the device, and an internal leakage channel extending between the fresh gas flow inlet and one of the patient interface end and the outlet.

According to another aspect of the present disclosure, there is provided a system for PPV and CPAP treatment comprising a device as disclosed herein, a fresh gas flow tube, a fresh gas source connected with the fresh gas flow inlet by means of the fresh gas flow tube, and a pressure release valve arranged to prevent an excessive positive pressure in a PPV mode. Thus, the system includes the fresh gas source, and a pressure release valve which is set to a specific opening pressure adapted to suit the patient to be treated.

In accordance with an embodiment of the system, it is arranged such that when the open end of the outlet of the device is occluded, the pressure will increase from the variable flow CPAP generator until an opening pressure of the pressure release valve is reached, which increase in pressure results in an inspiratory flow, whereby the pressure in the system will remain at the set PPV pressure until the occlusion is removed from the outlet, and when the occluded outlet is opened, the pressure will return to the set CPAP level, whereby the reduction in pressure leads to an expiratory flow.

In accordance with an embodiment of the system, it is arranged such that during spontaneous breathing, the patient flow and the fresh gas flow leaves the system through the variable flow CPAP generator keeping the positive pressure within the airway stable, by varying the flow that generates the CPAP, whereby the CPAP in the airway can be adjusted as needed.

In accordance with an embodiment of the system, the pressure release valve is connected with one of the patient interface end and the outlet of the device. These are two alternative ways of arranging the pressure release valve in the system, where the main aim is to arrange it in a position where the pressure of interest is measurable in a reliable way. Arranging the pressure release valve in connection with the outlet of the device allows for removal of the pressure release tube and the pressure release connection portion, providing a compact device and system. However, providing a pressure release connection portion and a pressure release tube, at which the pressure release valve is arranged, is as effective for the resuscitation treatment and may be a more familiar way of arranging the pressure release valve for a user.

In accordance with an embodiment of the system, it further comprises a pressure measuring device. Thereby, it is possible to easily monitor the operation of the system and make desirable adjustments of the fresh gas flow. Preferably, the pressure measuring device is connected close to the patient interface end of the device. In accordance with an embodiment of the system having a device comprising a pressure release connection portion, the pressure measuring device is connected to the patient interface end through the pressure release connection portion. Naturally, the main aim is to arrange the pressure measuring device in a position where the pressure of interest is measurable in a reliable and accurate way.

The different embodiments of the system can be summarized as a system comprising a device having a fresh gas flow inlet connected to a variable flow CPAP generator in turn connected to a patient interface end and an outlet of the device, and an internal leakage channel extending between the fresh gas flow inlet and one of the patient interface end and the outlet. The system further comprising a fresh gas flow tube connected at one end to the fresh gas flow inlet and at the other end to a fresh gas source for supplying a fresh gas flow to the device. A pressure release valve is further comprised in the system, being connected to one of the outlet and the patient interface end of the device. Optionally, the system comprises a pressure measuring device arranged close to the patient interface end of the device for precise and reliable measuring of the pressure thereat.

The device as disclosed herein can be used in PPV and CPAP treatment for neonatal resuscitation.

The system as disclosed herein can be used for PPV and CPAP treatment for neonatal resuscitation.

These and other aspects, and advantages of the disclosure will be apparent from and elucidated with reference to the embodiments described hereinafter.

### BRIEF DESCRIPTION OF THE DRAWINGS

The disclosure will now be described in more detail and with reference to the appended drawings. The drawings are included to provide a further understanding of the present disclosure and are incorporated in and are a part of this specification. Other embodiments of the present disclosure, and many of the intended advantages of the present invention, will be readily appreciated as they become better understood by reference to the following detailed description. The elements of the drawings are not necessarily to scale relative to each other. The same reference numerals designate corresponding similar parts.
Fig. 1a is a schematic cross-sectional illustration of an embodiment of the device according to the present disclosure;
Fig. 1b is a schematic cross-sectional illustration of another embodiment of the device according to the present disclosure;
Fig. 1c is a schematic cross-sectional illustration of yet another embodiment of the device according to the present disclosure;
Figs. 2a-c schematically illustrate embodiments of the system according to the present disclosure;
Figs. 3a-b schematically illustrate flows of the system shown in Fig. 2a during positive pressure disclosure; and
Fig. 4 schematically illustrates flows in the system shown in Fig. 2a during spontaneous ventilation.

### DESCRIPTION OF PREFERRED EMBODIMENTS

Examples of different embodiments of the present disclosure are provided. Common for the embodiments of the device for PPV and CPAP treatment described herein is that they comprise a fresh gas flow inlet 2, a patient interface end 3, an outlet 4 having an open end 5, a variable CPAP generator 6, and an internal leakage channel 9. Furthermore, the variable flow CPAP generator 6 comprises a first connection portion 10, a second connection portion 11, and a third connection portion 12. In all embodiments, the fresh gas flow inlet 2 is connected with the first connection portion 10 of the variable flow CPAP generator 6; the patient interface end 3 is connected with the second connection portion 11; and the third connection portion 12 is connected with the outlet 4 of the device. The outlet 4 is typically tubular and arranged as a protruding tube ending having a free open end 5. The patient interface end 3 of the device is connectable with any patient interface, such as for example a face mask, nasal prongs, or an endotracheal tube.

The first, second and third connection portions 10, 11, 12 of the variable flow CPAP generator 6 are generally tubular. In the embodiments shown herein, the second and third connection portions 11, 12 of the variable flow CPAP generator 6 are arranged at an angle to each other. Further, the first connection portion 10 is connected with the third connection portion 12 at an angle, and is arranged substantially coaxially with the second connection portion 11. Such a geometry of the variable flow CPAP generator 6 is advantageous as it provides a stable CPAP generated by the variable flow CPAP generator 6, and a low imposed work of breathing for the patient when in use. However, providing first, second and third connection portions 10, 11, 12 of the variable flow CPAP generator 6 having a different geometry is also conceivable within the concept of the present invention.

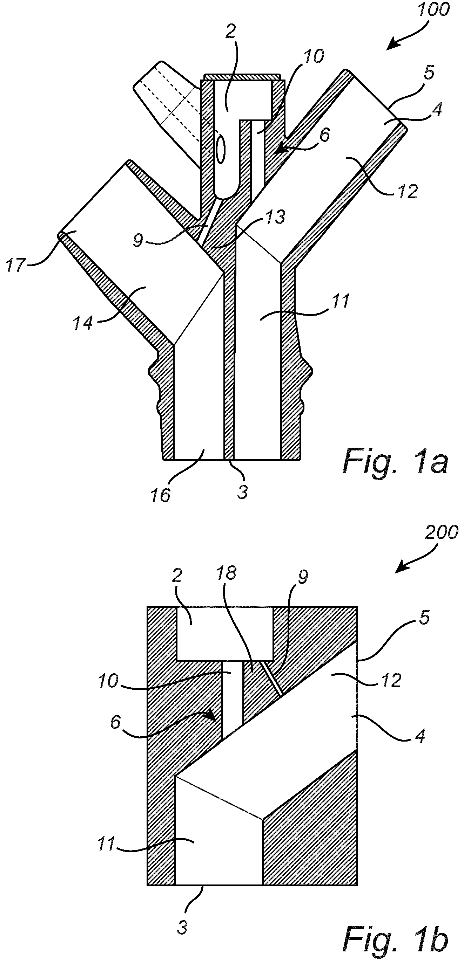

The internal leakage channel 9 may also be called internal bypass flow channel, since in the embodiments and operation modes the internal fresh gas leakage flow will bypass the first connection portion 10 of the variable CPAP generator 6 and will thereby not flow there through. The internal leakage channel 9 can be arranged in several different positions, see Figures 1a-c.

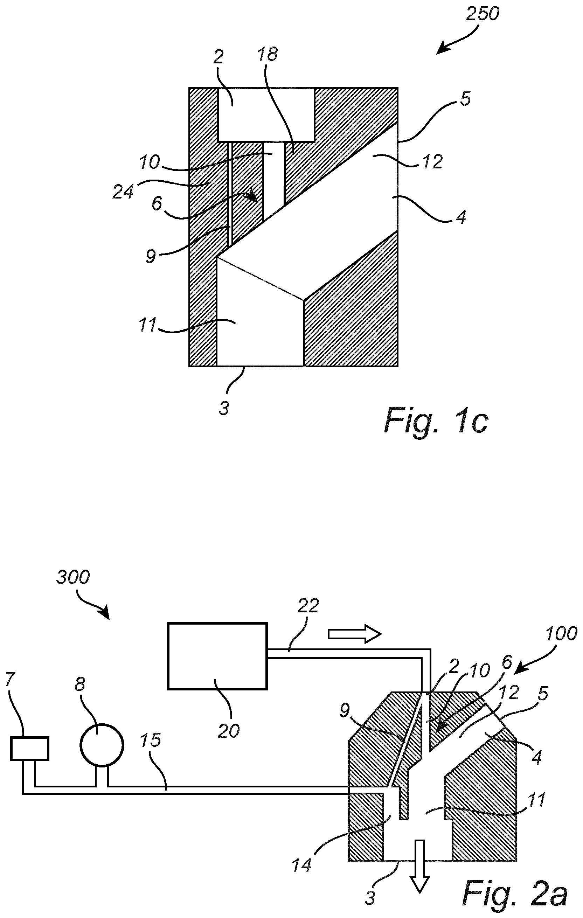

With reference to Figure 1a, according to a first embodiment of the device 100, the internal leakage channel 9 extends between the fresh gas flow inlet 2 and the patient interface end 3 through an internal wall portion 13 which extends between the fresh gas flow inlet 2 and a pressure release connection portion 14 of the device 100. The pressure release connection portion 14 is generally tubular forms a passage through a portion of the device 100. More particularly, the pressure release connection portion 14 extends partially in parallel with the second connection portion 11 of the variable flow CPAP generator 6 and comprises a first end 16 and a second end 17. The first end 16 is connected with the patient interface end 3 of the device 100 and the second end 17 is connectable to a pressure release tube 15, see Fig. 2a. Thus, the internal leakage channel 9 extends through the internal wall portion 13, which is present between the fresh gas flow inlet 2 and the pressure release connection portion 14, and further through the pressure release connection portion 14 to the patient interface end 3 of the device 100.

As disclosed in Figure 1b, according to a second embodiment of the device 200, the internal leakage channel 9 extends between the fresh gas flow inlet 2 and the outlet 4 of the device 200. In this exemplifying embodiment, the internal leakage channel 9 extends through the internal wall portion 18 which is present between fresh gas flow inlet 2 and the third connection portion 12 of the variable flow CPAP generator 6, and further through the third connection portion 12 to the outlet 4 of the device 200. Thus, the internal leakage channel 9 partially extends through the third connection portion 12 of the variable flow CPAP generator while bypassing the first connection portion 10. This provides a compact device 200.

According to yet another embodiment of the device 250, shown in Fig. 1c, the internal leakage channel 9 extends between the fresh gas flow inlet 2 and the patient interface end 3 of the device 250, partially through the second connection portion 11 of the variable flow CPAP generator 6. For this, a portion of the internal leakage channel 9 is provided as a hole through an internal wall portion 24 of the device 250 which extends between the fresh gas flow inlet 2 and the second connection portion 11 of the variable flow CPAP generator 6. Another portion of the internal leakage channel 9 thus extends through the second connection portion 11 to the patient interface end 3.

In the shown embodiments, the hole is substantially straight, and may be e.g. a bore. This allows adapting previous devices used for CPAP and PPV treatment, as that mentioned in the background of the invention, by providing a bore between a fresh gas flow inlet 2 and one of the patient interface end 3 and the outlet 4, bypassing the first connection portion 10 of the variable flow CPAP generator 6 and removing any second fresh gas flow tube. However, the skilled person will understand from the teaching of the present disclosure that providing an internal leakage channel 9 of any other shape or extension than those described herein with reference to the exemplifying embodiments is also possible within the concept of the present disclosure, provided that it extends between the fresh gas flow inlet 2 and one of the patient interface end 3 and the outlet 4, and bypasses the first connection portion 10 of the variable flow CPAP generator 6.

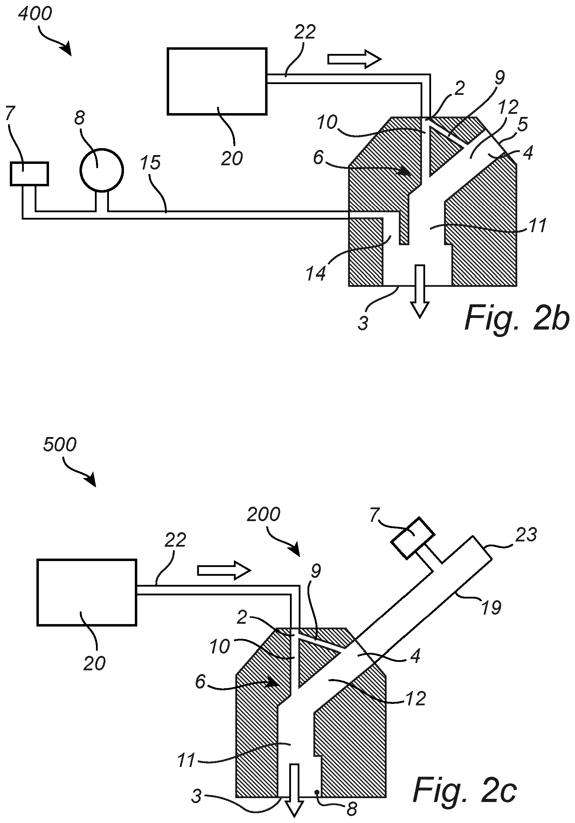

A first embodiment of the system 300, as disclosed in Figs. 2a, 3a-b and 4, comprises the embodiment of the device 100 described with reference to Fig. 1a. The system 300 further comprises a fresh gas source 20, which provides a fresh gas flow to the fresh gas flow inlet 2 through a fresh gas flow tube 22 of the system 300. The level of CPAP is adjusted at the fresh gas source 20. The system further comprises a pressure release valve 7 and a pressure measuring device 8. The pressure release valve 7 and the pressure measuring device 8 can be arranged in several different positions, see Figures 2 a-c. The pressure measuring device 8 can be omitted in any embodiment, however, if there is no interest in measuring the pressure. In the exemplifying embodiments shown in Figs. 2a and 2b, the system further comprises a pressure release tube 15 connected to the pressure release connection portion 14. The pressure release valve 7 and the pressure measuring device 8 are here connected to the pressure release connection portion 14 through the pressure release tube 15.

A second embodiment of the system 400, as disclosed in Fig. 2b, comprises an embodiment of the device comprising a pressure release connection portion 14, and in which the internal leakage channel 9 extends between the fresh gas flow inlet 2 and the outlet 4 of the device. The system 400 comprises a pressure release tube 15 connected to the pressure release connection portion 14. The pressure release valve 7 is arranged at an end of the pressure release tube 15 which is distal to the pressure release connection portion 14. The pressure measuring device 8 is arranged at the pressure release tube 15 between the pressure release valve 7 and the pressure release connection portion 14.

A third embodiment of the system 500, as disclosed in Fig. 2c, comprises the embodiment of the device 200 described with reference to Fig. 1b. In this exemplifying embodiment, the pressure release valve 7 is arranged in connection with the outlet 4 of the device 200. More particularly, the system 500 comprises an outlet tube 19 connected to the outlet 4 of the device 200 through its open end 5. The pressure release valve 7 is arranged along the outlet tube 19. The outlet tube 19 comprises an open end 23. Further, in this exemplifying embodiment, the pressure measuring device 8 is arranged close to the patient interface end 3 of the device 200.

The system is operated as follows. Reference will be made to the first embodiment of the system 300, but the second embodiment and third embodiments have a corresponding operation. Oxygen concentration and fresh gas flow are adjusted by a standard blender and a flow meter. The fresh gas flow can be varied and is typically set to between 5 and 15 litres per minute. More preferably, the fresh gas flow is set to between 10 and 12 litres per minute. A fresh gas flow in this range should prevent rebreathing, provide flow to achieve an adequate inspiration flow, volume and time, and provide some allowance for leakage in the patient interface, in addition to the internal leakage provided by the internal leakage channel 9.

The fresh gas flow is used to drive the variable flow CPAP generator 6. However, inside the device, a part of the fresh gas flows through the internal leakage channel 9 bypassing at least the first connection portion 10 of the variable flow CPAP generator 6. The internal bypass flow, i.e. the internal fresh gas leakage flow, is used to supply adequate flow for positive pressure ventilation by bypassing the variable flow CPAP generator 6. The internal bypass flow is connected in such a way that it adds to inspiratory patient flow when the device 1 is occluded during positive pressure ventilation. The fresh gas flow to the variable flow CPAP generator 6 is adjustable. A typical value of the fresh gas flow provided to the fresh gas flow inlet 2 is 5-15 liters per minute. To provide higher levels of CPAP, the flow driving the variable flow CPAP generator 6 can be increased further. For a same size of the internal leakage channel 9, i.e. for a same device 100, 200, a further increased driving flow generates a CPAP level that is higher than normally used. However, a device can be provided with an internal leakage channel 9 generating a greater leakage, e.g. by providing an increased diameter of the internal leakage channel 9, for which an increased drive flow is required to provide a certain level of CPAP. In the PPV mode, when occluding the open end 5 of the device 100, 200, the greater leakage and higher drive flow results in an increased flow added to the fresh gas flow generated by the variable flow CPAP generator 6, and thus to a faster increase in pressure.

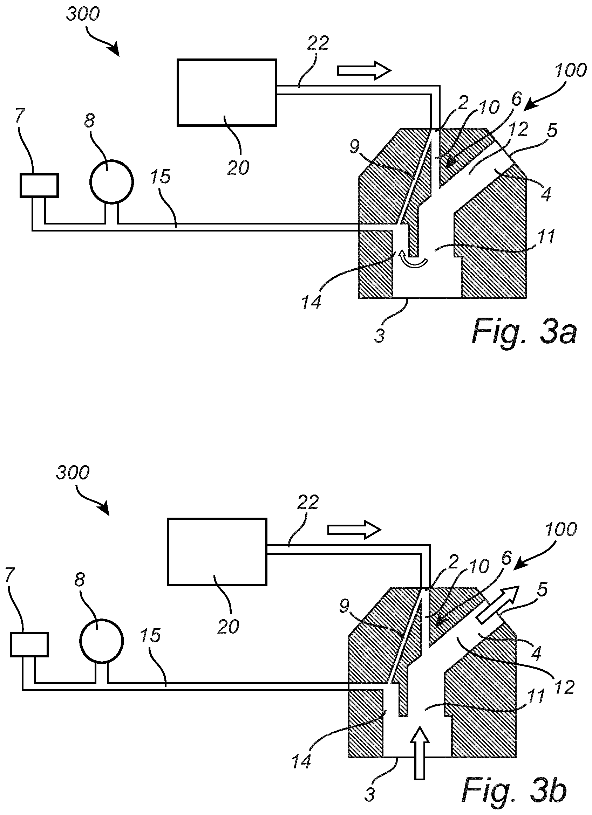

The outlet 4 of the variable flow CPAP generator 6 has an open end 5. If the open end 5 is occluded, see Figs. 2a and 3a, the pressure delivered to the infant will increase from the pressure set by the variable CPAP generator 6 until the opening pressure of the pressure release valve 7 is reached. A typical value for the pressure release valve 7 to open is around 20-30 cm H₂O. The increase in pressure results in an inspiratory flow. The pressure in the system 300 will remain at the set positive pressure ventilation pressure until the outlet occlusion is removed. When the occluded open end 5 is opened, see Figure 3b, the pressure delivered to the patient will return to the set CPAP level and this reduction in pressure will lead to an expiratory flow.

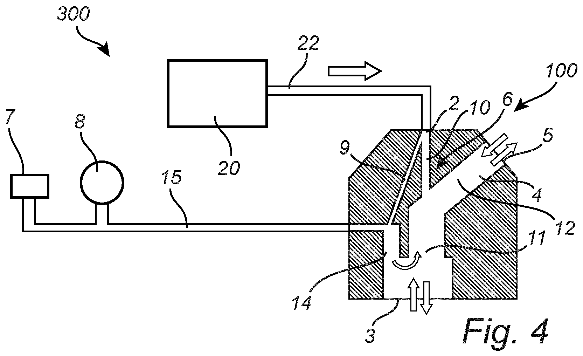

During spontaneous breathing the infant flow and the fresh gas flow leaves the system 300 through the variable flow CPAP generator 6, see Figure 4. This keeps the positive pressure within the airway stable. By varying the flow, i.e. the fresh gas flow, that generates the CPAP pressure, the CPAP pressure in the airway can be adjusted as needed.

The patient interface end 3 can be designed in any suitable form such to be suitable for connection with a patient interface. Further, the patient interface can assume a variety of designs suitable for establishing a connection to the patient nasal airways, not shown. Thus, the patient interface can include an opposing pair of nasal prongs, a mask, an endotracheal tube or any other suitable devices.

The system 300, 400, 500 could have a backup system for malfunctioning of the pressure release valve 7. This could either be an alarm, a second release valve or a system that cuts the fresh gas flow.

The pressure measuring device 8 should be positioned as close to the patient as possible to provide accurate recording of the pressure of the gas delivered to the patient. The accuracy will depend on the flow resistance of the patient interface and, for an infant, a low resistance interface should be used if possible. The embodiment of the device described with reference to Fig. 1b could be regarded as beneficial since there will be less tubes needed in the system 500.

Notably, the device, in accordance with principles of the present disclosure is useful with a wide variety of patient interface configurations that may or may not incorporate some or all of the features described above with respect to the patient interface. Thus, the patient interface is in no way limiting.

Notably, the device, in accordance with principles of the present disclosure is useful with a wide variety of variable flow CPAP generators that may or may not incorporate some or all of the features described above with respect to the variable flow CPAP generator 6. Thus, the model of the variable flow CPAP generator 6 is in no way limiting.

Notably, the device, in accordance with principles of the present disclosure is useful with a wide variety of pressure release valves 7 or similar devices that achieve the purpose of releasing air depending on the pressure in the system. Thus, the model or type of pressure release valve is in no way limiting.

Notably, the device, in accordance with principles of the present disclosure is useful with a wide variety of pressure measuring devices 8 or similar devices that achieves the purpose of measuring the pressure in the system. Thus, the model or type pressure measuring devices is in no way limiting.

A typical CPAP level for resuscitating or stabilising an infant is in the range of 3-10 cm H₂O. A typical peak pressure for PPV is 20-30 cm H₂O. It is obtained by occluding the systems and having a correctly adjusted pressure release valve.

Above embodiments of the device and system according to the present disclosure as defined in the appended claims have been described. These should only be seen as merely non-limiting examples. As understood by the person skilled in the art, many modifications and alternative embodiments are possible within the scope of the invention as defined by the appended claims. For example, according to an embodiment, the device comprises several variable flow CPAP generators, each being connected to the fresh gas flow inlet, the patient interface end and the outlet of the device. Together with the internal leakage channel, extending between the fresh flow gas inlet and at least one of the patient interface end and the outlet of the device, this allows operating the device at a high fresh gas flow while maintaining a stable level of CPAP in the CPAP mode. Further, in the PPV mode, the rise time, i.e. the time necessary for reaching a predetermined airway pressure of a patient when the open end of the outlet is occluded, is reduced.

According to another embodiment, the device comprises several internal leakage channels, each extending between the fresh gas flow inlet and one of the patient interface end and the outlet of the device. For example, a device may comprise first and second internal leakage channels wherein the first internal leakage channel extends between the fresh gas flow inlet and the patient interface end, and the second internal leakage channel extends between the fresh gas flow inlet and the outlet of the device. Providing several internal leakage channels also allows providing a higher fresh gas flow to the device while maintaining a stable CPAP level in the CPAP mode and decreasing the rise time in the PPV mode.

It is to be noted that for the purposes of his application, and in particular with regard to the appended claims, the word "comprising" does not exclude other elements or steps, and the word "a" or "an" does not exclude a plurality, which per se will be evident to a person skilled in the art.

## Claims

1. A device (100, 200, 250) for positive pressure ventilation (PPV) and continuous positive airway pressure (CPAP) treatment comprising:
- a fresh gas flow inlet (2), arranged to receive a fresh gas flow from a fresh gas flow tube connectable thereto;
- a patient interface end (3), arranged to be connected with a patient interface;
- an outlet (4) having an open end (5);
- a variable flow CPAP generator (6) comprising first, second and third connection portions (10, 11, 12), wherein the first connection portion is connected with the fresh gas flow inlet, the second connection portion is connected with the patient interface end, and the third connection portion is connected with the outlet, wherein the device is configured such that a CPAP level generated by the variable flow CPAP generator is adjustable by varying the fresh gas flow to the fresh gas flow inlet and thereby to the variable flow CPAP generator; and
- an internal leakage channel (9) arranged to extend between the fresh gas flow inlet and at least one of the patient interface end and the outlet, such to create an internal fresh gas leakage flow there between, bypassing the first connection portion of the variable flow CPAP generator, which internal fresh gas leakage flow is added to the fresh gas flow provided by the variable flow CPAP generator in the PPV mode,
**characterized in that**
the internal leakage channel comprises a hole extending through an internal wall portion, which extends between the fresh gas flow inlet and one of the second connection portion, the third connection portion, and a pressure release connection portion (14), arranged partially in parallel with the second connection portion, and having a first end (16) connected with the patient interface end and a second end (17) connectable with a pressure release tube (15).

2. The device (250) according to claim 1, wherein the internal leakage channel (9) partially extends through the second connection portion (11) of the variable flow CPAP generator (6) to the patient interface end (3) of the device.

3. The device (200) according to claim 1, wherein the internal leakage channel (9) partially extends through the third connection portion (12) of the variable flow CPAP generator (6) to the outlet (4) of the device.

4. The device (100) according to claim 1, wherein the internal leakage channel (9) partially extends through the pressure release connection portion (14) to the patient interface end of the device.

5. The device (100, 200, 250) according to any one of the preceding claims where the fresh gas flow provided to the variable flow CPAP generator (6) is 5-15 liters per minute.

6. A system (300, 400, 500) for PPV and CPAP treatment comprising a device (100, 200, 250) according to any one of claims 1-5, a fresh gas flow tube (22), a fresh gas source (20) connected with the fresh gas flow inlet (2) by means of the fresh gas flow tube, and a pressure release valve (7) arranged to prevent an excessive positive pressure in a PPV mode.

7. The system (300, 400, 500) according to claim 6, arranged such that when the open end (5) of the outlet (4) of the device (100, 200, 250) is occluded, the pressure will increase from the variable flow CPAP generator (6) until an opening pressure of the pressure release valve (7) is reached, which increase in pressure results in an inspiratory flow, whereby the pressure in the system will remain at the set PPV pressure until the occlusion is removed from the outlet, and when the occluded outlet is opened, the pressure will return to the set CPAP level, whereby the reduction in pressure leads to an expiratory flow.

8. The system (300, 400, 500) according to claim 6, arranged such that during spontaneous breathing, the infant flow and the fresh gas flow leaves the system through the variable flow CPAP generator (6) keeping the positive pressure within the airway stable, by varying the flow that generates the CPAP, whereby the CPAP in the airway can be adjusted as needed.

9. The system (300, 400, 500) according to any one of claims 6-8,
wherein the pressure release valve (7) is connected with one of the patient interface end (3) and the outlet (4) of the device (100, 200, 250).

10. The system (300, 400, 500) according to any one of claims 6-9, further comprising a pressure measuring device (8).

## Patentansprüche

1. Vorrichtung (100, 200, 250) zur Behandlung mit Überdruckbeatmung (PPV *- Positive Pressure Ventilation*) und mit kontinuierlichem positivem Atemwegsdruck (CPAP - *Continuous Positive Airway Pressure*), welche Folgendes umfasst:
- einen Frischgasflusseinlass (2), der zum Empfangen eines Frischgasflusses von einem daran anschließbaren Frischgasflussschlauch geeignet ist;
- ein Patientenschnittstellenende (3), das zum Anschließen an eine Patientenschnittstelle geeignet ist;
- einen Auslass (4) mit einem offenen Ende (5);
- einen CPAP-Generator mit variablem Durchfluss (6), der einen ersten, einen zweiten und einen dritten Verbindungsabschnitt (10, 11, 12) umfasst, wobei der erste Verbindungsabschnitt mit dem Frischgasflusseinlass verbunden ist, der zweite Verbindungsabschnitt mit dem Patientenschnittstellenende verbunden ist und der dritte Verbindungsabschnitt mit dem Auslass verbunden ist, wobei die Vorrichtung derart konfiguriert ist, dass ein durch den CPAP-Generator mit variablem Durchfluss erzeugtes CPAP-Niveau durch Variation des Frischgasflusses zum Frischgasflusseinlass und damit zum CPAP-Generator mit variablem Durchfluss anpassbar ist; und
- einen internen Leckagekanal (9), der so angeordnet ist, dass er sich zwischen dem Frischgasflusseinlass und mindestens entweder dem Patientenschnittstellenende oder dem Auslass erstreckt, sodass ein interner Frischgas-Leckagefluss dazwischen erzeugt wird, wodurch der erste Verbindungsabschnitt des CPAP-Generators mit variablem Durchfluss umgangen wird, wobei im PPV-Modus der interne Frischgas-Leckagefluss zu dem Frischgasfluss hinzugefügt wird, der durch den CPAP-Generator mit variablem Durchfluss bereitgestellt wird,
**dadurch gekennzeichnet, dass** der interne Leckagekanal ein Loch, das sich durch einen Innenwandabschnitt erstreckt, welches sich zwischen dem Frischgasflusseinlass und entweder dem zweiten Verbindungsabschnitt oder dem dritten Verbindungsabschnitt erstreckt, und einen Druckentlastungsverbindungsabschnitt (14), der teilweise parallel zum zweiten Verbindungsabschnitt angeordnet ist und ein erstes Ende (16), das mit dem Patientenschnittstellenende verbunden ist, und ein zweites Ende (17), das mit einem Druckentlastungsschlauch (15) verbunden werden kann, aufweist, umfasst.

2. Vorrichtung (250) nach Anspruch 1, wobei sich der interne Leckagekanal (9) teilweise durch den zweiten Verbindungsabschnitt (11) des CPAP-Generators mit variablem Durchfluss (6) zum Patientenschnittstellenende (3) der Vorrichtung erstreckt.

3. Vorrichtung (200) nach Anspruch 1, wobei sich der interne Leckagekanal (9) teilweise durch den dritten Verbindungsabschnitt (12) des CPAP-Generators mit variablem Durchfluss (6) zum Auslass (4) der Vorrichtung erstreckt.

4. Vorrichtung (100) nach Anspruch 1, wobei sich der interne Leckagekanal (9) teilweise durch den Druckentlastungsverbindungsabschnitt (14) zum Patientenschnittstellenende der Vorrichtung erstreckt.

5. Vorrichtung (100, 200, 250) nach einem der vorhergehenden Ansprüche, wobei der an den CPAP-Generator mit variablem Durchfluss (6) bereitgestellte Frischgasfluss 5-15 Liter pro Minute beträgt.

6. System (300, 400, 500) zur PPV- und CPAP-Behandlung, welches eine Vorrichtung (100, 200, 250) nach einem der Ansprüche 1-5, einen Frischgasflussschlauch (22), eine Frischgasquelle (20), die mithilfe des Frischgasflussschlauchs mit dem Frischgasflusseinlass (2) verbunden ist, und ein Druckentlastungsventil (7), das zum Verhindern eines übermäßigen Überdrucks in einem PPV-Modus geeignet ist, umfasst.

7. System (300, 400, 500) nach Anspruch 6, welches derart angeordnet ist, dass, wenn das offene Ende (5) des Auslasses (4) der Vorrichtung (100, 200, 250) verschlossen ist, der Druck vom CPAP-Generator mit variablem Durchfluss (6) ansteigt, bis ein Öffnungsdruck des Druckentlastungsventils (7) erreicht ist, wobei dieser Druckanstieg in einem Einatemfluss resultiert, wodurch der Druck im System auf dem eingestellten PPV-Druck verbleibt, bis der Verschluss vom Auslass entfernt wird, und, wenn der verschlossene Auslass geöffnet wird, der Druck auf das eingestellte CPAP-Niveau zurückkehrt, wodurch die Druckminderung zu einem Ausatemfluss führt.

8. System (300, 400, 500) nach Anspruch 6, welches derart angeordnet ist, dass bei Spontanatmung der Säuglingsfluss und der Frischgasfluss das System durch den CPAP-Generator mit variablem Durchfluss (6) verlassen, wodurch der Überdruck innerhalb der Atemwege stabil gehalten wird, indem der Fluss, der den CPAP erzeugt, variiert wird, wodurch der CPAP in den Atemwegen nach Bedarf angepasst werden kann.

9. System (300, 400, 500) nach einem der Ansprüche 6-8, wobei das Druckentlastungsventil (7) entweder mit dem Patientenschnittstellenende (3) oder dem Auslass (4) der Vorrichtung (100, 200, 250) verbunden ist.

10. System (300, 400, 500) nach einem der Ansprüche 6-9, welches ferner eine Druckmessvorrichtung (8) umfasst.

## Revendications

1. Dispositif (100, 200, 250) pour un traitement par ventilation en pression positive (PPV) et par ventilation en pression positive continue (CPAP) comprenant :
- une entrée de circulation de gaz neuf (2) agencée pour recevoir une circulation de gaz neuf d'un tube de circulation de gaz neuf pouvant être connecté dessus ;
- une extrémité d'interface patient (3) agencée pour être connectée à une interface patient ;
- une sortie (4) ayant une extrémité ouverte (5) ;
- un générateur de CPAP à circulation variable (6) comprenant des première, deuxième et troisième parties de connexion (10, 11, 12), dans lequel la première partie de connexion est connectée à l'entrée de circulation de gaz neuf, la deuxième partie de connexion est connectée à l'extrémité d'interface patient, et la troisième partie de connexion est connectée à la sortie, dans lequel le dispositif est configuré de telle façon qu'un niveau de CPAP généré par le générateur de CPAP à circulation variable est ajustable en faisant varier la circulation de gaz neuf vers l'entrée de circulation de gaz neuf et ainsi vers le générateur de CPAP à circulation variable ; et
- un canal de fuite interne (9) agencé pour s'étendre entre l'entrée de circulation de gaz neuf et au moins une de l'extrémité d'interface patient et de la sortie, de façon à créer une circulation de fuite de gaz neuf interne entre eux, dérivant la première partie de connexion du générateur de CPAP à circulation variable, laquelle circulation de fuite de gaz neuf interne est ajoutée à la circulation de gaz neuf fournie par le générateur de CPAP à circulation variable dans le mode PPV,
**caractérisé en ce que**
le canal de fuite interne comprend un trou s'étendant à travers une partie de paroi intérieure, laquelle s'étend entre l'entrée de circulation de gaz neuf et une de la deuxième partie de connexion, la troisième partie de connexion et une partie de connexion de libération de pression (14), agencée partiellement en parallèle avec la deuxième partie de connexion, et ayant une première extrémité (16) connectée à l'extrémité d'interface patient et une deuxième extrémité (17) pouvant être connectée à un tube de libération de pression (15).

2. Dispositif (250) selon la revendication 1, dans lequel le canal de fuite interne (9) s'étend partiellement à travers la deuxième partie de connexion (11) du générateur de CPAP à circulation variable (6) vers l'extrémité d'interface patient (3) du dispositif.

3. Dispositif (200) selon la revendication 1, dans lequel le canal de fuite interne (9) s'étend partiellement à travers la troisième partie de connexion (12) du générateur de CPAP à circulation variable (6) vers la sortie (4) du dispositif.

4. Dispositif (100) selon la revendication 1, dans lequel le canal de fuite interne (9) s'étend partiellement à travers la partie de connexion de libération de pression (14) vers l'extrémité d'interface patient du dispositif.

5. Dispositif (100, 200, 250) selon l'une quelconque des revendications précédentes, où la circulation de gaz neuf fournie au générateur de CPAP à circulation variable (6) est de 5-15 litres par minute.

6. Système (300, 400, 500) pour le traitement par PPV et CPAP comprenant un dispositif (100, 200, 250) selon l'une quelconque des revendications 1-5, un tube de circulation de gaz neuf (22), une source de gaz neuf (20) connectée à l'entrée de circulation de gaz neuf (2) au moyen du tube de circulation de gaz neuf, et une soupape de libération de pression (7) agencée pour empêcher une pression positive en excès dans un mode de PPV.

7. Système (300, 400, 500) selon la revendication 6, agencé de telle façon que lorsque l'extrémité ouverte (5) de la sortie (4) du dispositif (100, 200, 250) est fermée, la pression augmentera du générateur de CPAP à circulation variable (6) jusqu'à ce qu'une pression d'ouverture de la soupape de libération de pression (7) soit atteinte, laquelle augmentation de pression résulte en un flux inspiratoire, ce par quoi la pression dans le système restera à la pression de PPV réglée jusqu'à ce que l'occlusion soit retirée de la sortie, et lorsque la sortie occluse sera ouverte, la pression retournera au niveau de CPAP réglé, ce par quoi la réduction de pression conduit à un flux expiratoire.

8. Système (300, 400, 500) selon la revendication 6, agencé de telle façon que pendant la respiration spontanée, le flux infantile et la circulation de gaz neuf quittent le système à travers le générateur de CPAP à circulation variable (6), maintenant la pression positive à l'intérieur de la ventilation stable, en faisant varier la circulation qui génère la CPAP, ce par quoi la CPAP dans la ventilation peut être ajustée selon les besoins.

9. Système (300, 400, 500) selon l'une quelconque des revendications 6-8, dans lequel la soupape de libération de pression (7) est connectée à l'une de l'extrémité d'interface patient (3) et de la sortie (4) du dispositif (100, 200, 250).

10. Système (300, 400, 500) selon l'une quelconque des revendications 6-9, comprenant en outre un dispositif de mesure de pression (8).
